(19) Europäisches Patentamt — European Patent Office — Office européen des brevets

(11) **EP 4 249 008 B1**

(12) **EUROPEAN PATENT SPECIFICATION**

(45) Date of publication and mention of the grant of the patent:
**18.09.2024 Bulletin 2024/38**

(21) Application number: **23163298.5**

(22) Date of filing: **21.03.2023**

(51) International Patent Classification (IPC):
**A61L 27/24** (2006.01)   **A61L 27/50** (2006.01)
**A61L 27/52** (2006.01)   **A61L 27/54** (2006.01)
**B33Y 80/00** (2015.01)

(52) Cooperative Patent Classification (CPC):
**A61L 27/24; A61L 27/50; A61L 27/52; A61L 27/54; B33Y 80/00;** A61L 2400/06

(54) **BIOINK COMPOSITION BASED ON EQUINE TYPE I COLLAGEN, PROCESS FOR ITS PREPARATION AND ITS USES**

BIOINK-ZUSAMMENSETZUNG AUF DER BASIS VON PFERDEKOLLAGEN TYP I, VERFAHREN ZU IHRER HERSTELLUNG UND IHRE VERWENDUNG

COMPOSITION DE BIOENCRE À BASE DE COLLAGÈNE ÉQUIN DE TYPE I, SON PROCÉDÉ DE PRÉPARATION ET SES UTILISATIONS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.03.2022 IT 202200005534**

(43) Date of publication of application:
**27.09.2023 Bulletin 2023/39**

(73) Proprietor: **Typeone Biomaterials S.r.l.**
**73021 Calimera (LE) (IT)**

(72) Inventors:
• **SALVATORE, Luca**
**73021 Calimera (LE) (IT)**
• **NATALI, Maria Lucia**
**73021 Calimera (LE) (IT)**

• **GALLO, Nunzia**
**73021 Calimera (LE) (IT)**

(74) Representative: **Gislon, Gabriele**
**Marietti, Gislon e Trupiano S.r.l.**
**Via Larga, 16**
**20122 Milano (IT)**

(56) References cited:
**WO-A1-2018/225076     WO-A1-93/02639**

• **NOCERA ADEN DÍAZ ET AL: "Development of 3D printed fibrillar collagen scaffold for tissue engineering", BIOMEDICAL MICRODEVICES, SPRINGER US, NEW YORK, vol. 20, no. 2, 27 February 2018 (2018-02-27), pages 1 - 13, XP036441294, ISSN: 1387-2176, [retrieved on 20180227], DOI: 10.1007/S10544-018-0270-Z**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Summary of the invention

Subject-matter of the present invention is a bioink composition based on equine type I collagen, a process for its preparation and its uses in therapy and surgery.

## Technical background

3D printing technology enables high-precision creation of three-dimensional constructs with variable and customizable geometric complexities. Recently, much attention has been paid to 3D printing for the production of functional tissues/organs for biomedical applications and for *in vitro* cell modeling. The ability to produce implantable medical devices that may be customized to each patient's specific needs in terms of anatomical structure and pathology/trauma makes 3D printing unquestionably beneficial. Furthermore, the ability to reproduce *in vitro* a human tissue in its native three-dimensional conformation enables the creation of a cellular model that is able to more closely reflect the complex real-world cellular responses than 2D cultures and animal models. Thus, 3D printing holds a prominent place among all scaffold production methods as it has the potential to bridge the divergence between engineered constructs and native tissues.

There are currently two approaches for producing 3D constructs: *in vitro* and *in situ.* In the case of *in vitro* 3D printing, material deposition takes place in laboratory setups. Only after printing the construct is used for *in vitro* cell studies or for implantation in an animal model. In the case of *in situ* 3D printing, the material deposition is carried out directly at the site of interest of the animal and/or human model.

Regardless of the 3D printing approach there are different types of techniques, of which the most popular are those inkjet-based, light-assisted and extrusion-based. Inkjet printing (the first type of printing developed) uses an inkjet printer capable of spreading micro-droplets of cells and biomaterials by thermal, piezoelectric or acoustic means. Light energy-mediated printing is based on the application of light energy to induce the polymerization of a special light-polymerizable ink and may be subdivided into three types: LIFT (laser-induced forward transfer), SLA (stereo-lithography) and DLP (digital light projector). Extrusion printing allows 3D structures to be generated by extrusion deposition of ink through a nozzle on a support by applying pressure. Although new printing techniques have been developed (e.g., contactless printing and volumetric printing), the extrusion remains the most widely used technique in both research and commercial areas because it is extremely cheap, accessible and allows the complexity of biological tissues to be replicated quite closely. The main advantages of extrusion printing, compared with other techniques, are due to the ability to use a wide variety of biomaterials and cell types, to successfully deposit biomaterials with the desired cell density and to reduce the cell damage induced by the printing process compared with other techniques. Although it has some limitations, such as the range of available bioinks and filament resolution (typically greater than 100 $\mu$m), the advantages associated with the economic aspects and commercial availability have made the extrusion the most popular technique among engineers and researchers. Common bioinks include alginate hydrogels, collagen, gelatin, cellulose, fibroin and decellularized extracellular matrix. Among various bioinks, collagen is regarded as the biomaterial of choice. Most collagen-based inks are constituted by type I collagen, a protein with primarily structural function that constitutes about 90% of the protein mass of the mammalian connective tissues. Type I collagen bioinks, mostly of the "soluble" type and isolated from rat tail tendon or bovine/pig dermis, consist of acidic aqueous suspensions of collagen that under physiological conditions (neutral pH, 37°C) gel due to the ability of the collagen molecules to self-organize into fibrils. The printability of a collagen-based bioink depends on the kinetics of this process (gelling): the higher the speed, the higher the printing accuracy. Unfortunately, modulating such kinetics is not simple. Furthermore, the narrow time window and complete instability over time make the exploitation of this property not always optimal and adjustable. Some formulations, on the other hand, exhibit excessively slow gelling kinetics so that the extruded material does not hold its shape and the final 3D construct does not have high definition. In addition to these issues, collagen-based bioinks have been shown to have poor printability due to low mechanical properties. In order to implement the properties and printability of the collagen-based formulations, several strategies have been developed such as the hybridization with other materials/polymers (e.g., glycosaminoglycans, tricalcium phosphates, polyglycerol methacrylate), exposure to physical (e.g., UV light, heat) or chemical crosslinkers (e.g., by the use of coaxial nozzles that simultaneously provide bioink and crosslinking solution), and processing at low temperatures or high polymer concentrations. Processing at low temperatures allows the viscosity of the bioink to be increased and consequently solid structures to be printed. However, in addition to the fact that some formulations with low biopolymer concentration remain in the liquid state even at low temperatures, such approaches may compromise the printing of advanced structures with bioactive cells or molecules. In fact, unlike cell-free approaches that are successfully achieved by working at low temperatures, cell-loaded approaches require a temperature of about 37°C (as well as neutral pH). Parallel to the studies based on lowering the temperature, a considerable amount of work has sought to improve the printability of collagen suspensions by improving its storage modulus (or G'), as the behavior of the material is highly dependent on such parameter. In particular, a polymer suspension tends to behave as an elastic solid when the storage modulus G' is significantly greater than the loss modulus G", making it particularly suitable for use in printing processes, while on the other hand it exhibits a fundamentally viscous fluid-like behavior when it is characterized by a loss component greater than the storage one, making it unsuitable for printing in the latter case. The storage modulus depends on the concentration of salts in the suspension, on the temperature and collagen concentration. The most effective method to increase the storage modulus is to increase the concentration of collagen. Usually, collagen suspensions are low in concentration, usually no more than 5 mg/ml and rarely 10 mg/ml (more than 90% of known studies have been carried out by using collagen hydrogels prepared from solutions with no more than 10 mg/ml collagen). Only high concentrations of collagen (20 mg/ml or higher) allow a much higher storage modulus than the loss modulus and thus the printing accuracy to be increased.

In addition to the aforementioned approaches, a new technology called FRESH (Freeform Reversible Embedding of Suspended Hydrogels) has recently been developed that allows the use of acidic collagen suspensions having low viscosity for the development of 3D constructs by instantaneous gelling in an appropriate neutralization buffer and thus allows the availability of collagen-based bioinks to be expanded.

Therefore, it is understood that known collagen-based bioinks are constituted by acidic suspensions of collagen at low concentrations, usually around 0.5% and in rare cases up to 1%, which must then be neutralized *in situ* by using specific buffer solutions or require terminal treatments for post-printing crosslinking to definitely stabilize their structure.

NOCERA ADEN DIAZ ET AL: "Development of 3D printed fibrillar collagen scaffold for tissue engineering",BIOMEDICAL MICRODEVICES, SPRINGER US, NEW YORK, vol. 20, no. 2, 27 February 2018 (2018-02-27), pages 1-13, discloses a scaffold printed using a bovine collagen type-I bioink having shear tinning properties at a concentration of 6% w/v, pH 7 in PBS. The printed scaffold does not require crosslinking.

Therefore, there is a need for novel collagen-based compositions that may be used as bioinks that are suitable for the *in vitro* and *in situ* production of 3D constructs and are compatible with a number of printing systems and technologies that overcome the drawbacks of the known art and are stable in a physiological environment.

## Objects of the invention

A first object of the invention is to provide a collagen bioink composition suitable for the *in vitro* and *in situ* production of 3D constructs and compatible with conventional printing systems and technologies.

Another object of the invention is to provide a bioink composition that has neutral pH, an optimal condition for cell inclusion prior to printing, and specific chemical and physical properties thanks to which it is stable over a wide temperature range, particularly at 37°C, and does not need buffer solutions for gelling.

Another object of the invention is to provide a bioink composition that contains a collagen characterized by high preservation of its native hierarchical organization. Another object of the invention is to provide a bioink composition characterized by optimal rheological properties for use in 3D printing applications with extrusion technology.

Another object of the invention is to provide a process for preparing the collagen contained in the bioink composition of the invention, as well as the collagen thus obtained.

Finally, a further object of the invention is directed to the composition of the invention for use in therapy and surgery.

Such objects are achieved by the present invention, which provides a collagen bioink composition according to claim 1, a process for its preparation according to claim 8, a composition according to claim 12 and a 3D printing method according to claim 13.

## Brief description of the Figures

Figure 1 shows the flowchart of the production process of the bioink composition of the invention.

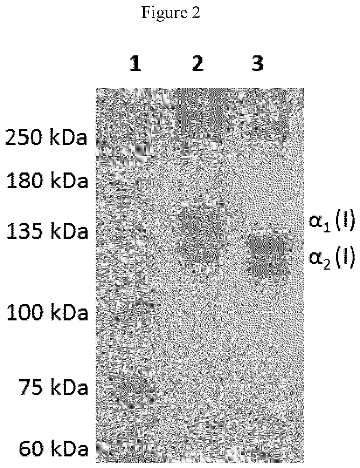

Figure 2 shows the electrophoretic pattern of the bioink composition of the invention (column 2) compared with native type I collagen (column 3). In column 1 proteins with known molecular weights between 10 and 250 kDa as reference standards are shown.

Figure 3 shows the banding pattern of the bioink composition of the invention on the left. The banding pattern of the native type I collagen is depicted on the right.

Figure 4 shows the Zeta Potential of the bioink composition of the invention (black line) compared with native type I collagen (gray line), as a function of pH (3.5<pH <4.5).

Figure 5 shows on the left the viscosity trend of the bioink composition of the invention (black line) compared to native type I collagen (gray line), where one may see the typical pseudoplastic "shear-thinning" behavior of the bioink itself, which may be modeled according to the power law or Ostwald-de Waele equation

$$\eta = \frac{\tau}{\dot{\gamma}} = m(\dot{\gamma})^{n-1}$$

with n<1. The storage modulus G' (solid black line) and loss modulus G" (dashed black line) of the invention and the storage modulus G' (solid gray line) and loss modulus G" (dashed gray line) of the native type I collagen are depicted on the right.

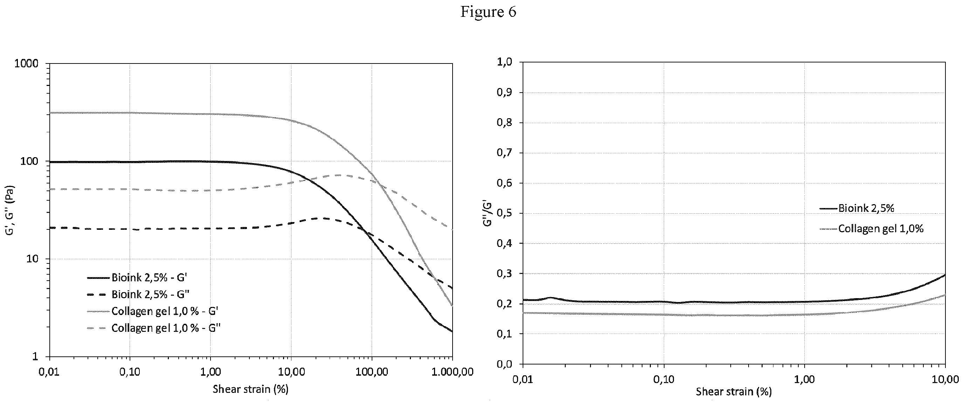

Figure 6 shows on the left the storage modulus G' (solid black line) and loss modulus G" (dashed black line) of the invention and the storage modulus G' (solid gray line) and loss modulus G" (dashed gray line) of the native type I collagen as a function of shear stress. On the right the G"/G' loss tangent of the invention (solid black line) and that of the native type I collagen (gray line) are depicted.

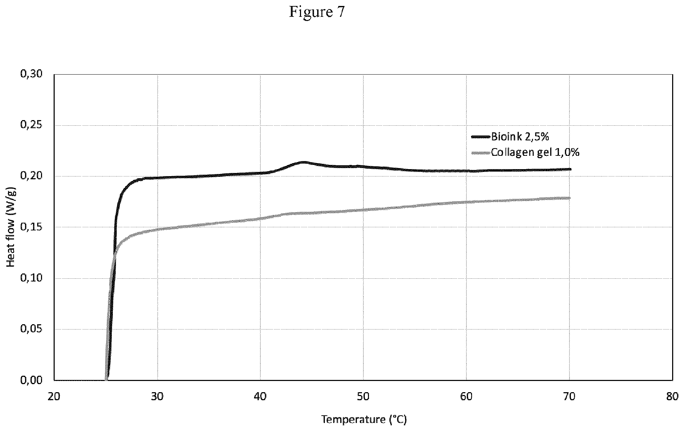

Figure 7 shows the thermogram of the bioink composition of the invention (black line) compared with the native type I collagen (gray line), where it may be seen that the material is thermally stable up to 40-42°C, i.e., up to this temperature no endo/exothermic phenomenon occurs upon heating.

## Description of the invention

According to one of its aspects, subject-matter of the invention is a bioink composition comprising type I equine collagen, characterized in that

a. said equine type I collagen is in suspension and has a weight average molecular weight of the $\alpha_1$(I) and $\alpha_2$(I) chains ranging from 120 to 200 kDa;
b. pH of said composition is from 7.0 to 7.6;
c. said composition is pseudoplastic.

Preferably, said equine type I collagen is in fibrillar form and exhibits the typical banding pattern of the native collagen with a period of 67 nm.

According to a preferred embodiment, the composition of the invention is an aqueous suspension in which the collagen is suspended in a liquid selected from a pharmaceutically acceptable buffer, preferably a phosphate buffered saline, and a physiological solution 0.9% w/v sodium chloride.

According to a preferred embodiment, the composition of the invention is an aqueous suspension in which the collagen is suspended at a concentration of 0.5 to 4.0% w/v, preferably 1.0 to 3.0% w/v.

According to a preferred embodiment, the composition of the invention has a viscosity η having values between 1 and 10000 Pa*s, for strain rates of 0.1 Hz, and between 0.01 and 100 Pa*s for strain rates of 100 Hz. Preferably, the composition of the invention has a viscosity η having values between 5 and 5000 Pa*s, for strain rates of 0.1 Hz, and between 0.1 and 10 Pa*s for strain rates of 100 Hz.

According to a preferred embodiment, the composition of the invention has storage modulus G' greater than the loss modulus G". According to a preferred embodiment, the composition of the invention has storage modulus G' having values between 1 and 10000 Pa, for frequencies of 0.1 Hz, and between 10 and 10000 Pa for frequencies of 10 Hz, and loss modulus G" having values between 0.1 and 1000 Pa, for frequencies of 0.1 Hz, and between 1 and 1000 Pa for frequencies of 10 Hz. Preferably, the composition of the invention has storage modulus G' having values between 5 and 5000 Pa, for frequencies of 0.1 Hz, and between 50 and 5000 Pa for frequencies of 10 Hz, and loss modulus G" having values between 0.5 and 500 Pa, for frequencies of 0.1 Hz, and between 5 and 500 Pa for frequencies of 10 Hz.

According to a preferred embodiment, the composition of the invention has loss tangent (G"/G') between 0.05 and 0.5, for shear stresses between 0.01% and 10%. Preferably, the composition of the invention has loss tangent having values between 0.1 and 0.3 for shear stresses between 0.01% and 10%.

More specifically, it was observed that it is advantageous for there to be a balance between the two components since the material must be printable (i.e., it must flow through the nozzle) and at the same time it must retain its shape after printing. A good compromise between the two components is obtained when the G"/G' ratio (or loss tangent) is about 0.1÷0.3.

The term "pseudoplastic" is well known to the skilled in the art and is often referred to by the expression "shear-thinning".

According to a preferred embodiment, the bioink composition comprising equine type I collagen is characterized in that

- said composition has G' > G" and G"/G' ratio preferably between 0.1 and 0.3;
- pH of said composition is from 7.0 to 7.6;
- said composition is pseudoplastic;

- said equine type I collagen is in suspension and has a weight average molecular weight of the $\alpha_1$(I) and $\alpha_2$(I) chains ranging from 120 to 200 kDa; and
- said equine type I collagen is in fibrillar form and exhibits the typical banding pattern of the native collagen with a period of 67 nm.

In contrast to the known collagen bioink compositions, the composition of the invention is constituted only by type I equine collagen, where "constituted only by type I equine collagen" means that it does not require additives, neutralizing agents, crosslinking agents and the like, which are conversely required for the stabilization and printing of the bioink compositions of the prior art. Furthermore, and most importantly, the composition of the invention does not need to be mixed with other copolymers (or base materials) in order to improve its performance; however, although it is not necessary, if desired such mixing may still be accomplished.

Before being subjected to printing, the composition of the invention may be loaded with components useful for its intended use.

According to an embodiment, the composition of the invention also comprises at least one other biological agent, where the term "biological agent" includes, but is not limited to, therapeutic agents, drugs, growth factors, proteins, cells and mixtures thereof. In general, any "adherent" cell type or cell mixture belonging to an immortalized or primary line (comprising pluripotent, multipotent. unipotent stem cells and differentiated cells) may be included in the composition of the invention, thanks to the fact that said composition has suitable properties to support and promote cellular processes and does not need exogenous treatments - such as, for example, UV irradiation or the addition of harmful/toxic additives/cross-linking agents - that would cause alteration/degradation of the loaded molecules and/or cell suffering or death.

By way of example, the bioink composition of the invention has been successfully printed with endothelial cells, fibroblasts, mixture of endothelial cells and fibroblasts, and myoblasts.

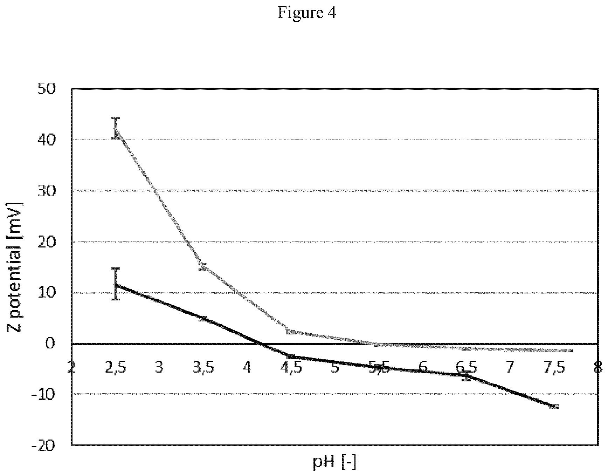

According to the invention, the type I equine collagen (herein below also just "collagen") is acylated and has free carboxyl groups. According to an embodiment, the collagen is acylated by reaction with a cyclic anhydride, preferably, but not only, succinic anhydride or glutaric anhydride. The acylation reaction with cyclic anhydrides converts to amide groups the basic $\varepsilon$-amine groups of the lysine residues in the collagen and provides free acid groups. It has been observed that by altering the net charge of the protein (a positive charge is replaced with a unit of negative charge) makes the collagen poly-anionic at physiological pH, that is, it shifts its isoelectric point, in this specific case, from a value of about 5.5 to a value between 3.5 and 4.5 (see Figure 4).

The purpose for which the acylation with cyclic anhydrides is carried out is, precisely, to make the collagen poly-anionic at physiological pH; this allows homogeneous and stable collagen suspensions to be made at such pH values. However, at the same time, it may be important for the collagen to retain free amine groups available for possible additional post-synthesis cross-linking. Therefore, although an amount of cyclic anhydride in excess of the theoretical number of free amines is preferably used for acylation, preferably the acylation reaction must not get to completeness. The skilled in the art is absolutely capable of managing the reaction to achieve this result.

According to a preferred embodiment, the composition of the invention is characterized by

- neutral pH, an optimal condition for the inclusion of cells before printing;
- specific chemical and physical properties, thanks to which it is stable over a wide temperature range (for example, at 37°C, another optimal condition for working with cells) and does not need buffer solutions (for gelling to take place);
- optimal rheological properties for 3D printing (especially for extrusion technology). Thus, thanks to the properties of the collagen acquired by the acylation treatment described above with cyclic anhydrides, as well as the other properties previously listed of the composition, it is possible to directly print the composition of the invention at neutral pH and body temperature and without the need for a neutralization buffer, additives or any chemical crosslinkers. This feature of the collagen also allows the inclusion in the composition of the invention of cells or molecular factors that may be printed without being damaged.

The composition of the invention is also characterized by optimal rheological properties that make it compatible with the more widespread bioprinting technologies and, preferably, extrusion printing systems.

According to a preferred embodiment, the 3D printing technology for the composition of the invention is based on extrusion processes. It has been observed that the deposition may be successfully carried out for temperatures between 4°C and 37°C, preferably with a needle extruder, for example, having a diameter of 0.15-1.20 mm, and with extrusion speeds, for example, from 5 to 50 mm/minute. However, the conditions in terms of flow/speed given above may be customized based on the viscosity of the formulation, which may be changed according to the application for which it is intended. The skilled in the art is certainly able to select the best 3D printing conditions from the information provided herein and his general knowledge.

The composition of the invention is a viscoelastic material, more precisely a non-Newtonian fluid of the shear-thinning or pseudoplastic type (its - apparent - viscosity decreases as strain rate increases). Thus, its behavior may be

$$\eta = \frac{\tau}{\dot{\gamma}} = m(\dot{\gamma})^{n-1}$$

described by a "power-law" type or Ostwald-de Waele equation                      with n<1. Therefore, the most important parameters from the rheological and dynamic-mechanical point of view for material characterization are the viscosity and G" (storage modulus, representing the elastic component of the material) and G" (loss modulus, representing the viscous component of the material) moduli. Such parameters are measured in a way known in the art, for example, by dynamic-mechanical analysis with the aid of a parallel-plate rheometer. The measurement of such parameters was carried out by a parallel-plate rotational rheometer with a plate diameter of 2.5 cm (StressTech HR High Resolution Oscillatory Rheometer - REOLOGICA Instruments). The test was performed at 25°C, after the sample reached the stated temperature, i.e., after a 5-minute pre-conditioning, with constant strain of 1%, varying the frequency from 0.1 to 100 Hz, for a duration of 12 minutes.

Other useful information for measuring the viscosity and G' and G" moduli may be found in the ISO 15798/2022 standard and on page 10 of the scientific publication "Guidelines for standardization of bioprinting: a systematic study of process parameters and their effect on bioprinted structures," under "Rheology" paragraph.

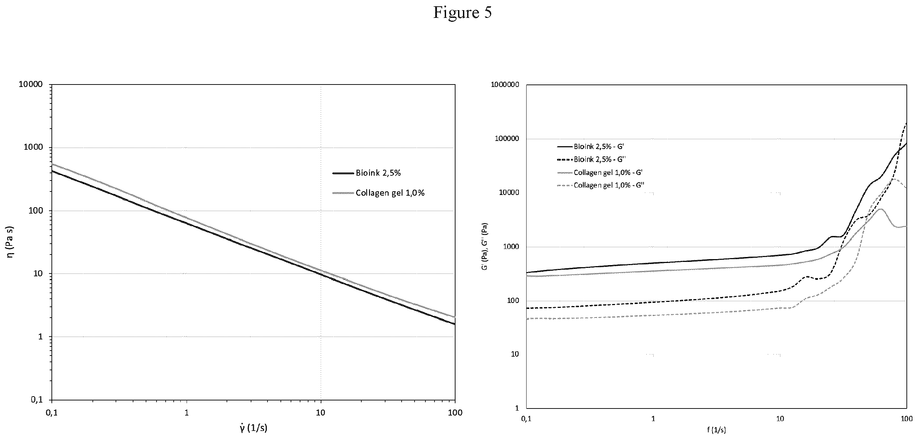

Figure 5 shows two representative graphs of the rheological properties of the composition (in black) compared to a standard preparation of native collagen (in gray). In particular, the graph on the left shows the trend of viscosity as a function of strain rate (from which the typical shear-thinning behavior of the two materials may be seen), the one on the right shows the trend of G' and G" moduli as a function of frequency. Figure 6 shows two representative graphs of the rheological properties of the composition (in black) compared to a standard preparation of native collagen (in gray). In particular, the graph on the left shows the trend of the G' and G" moduli as a function of shear stress, the one on the left shows the trend of the loss tangent.

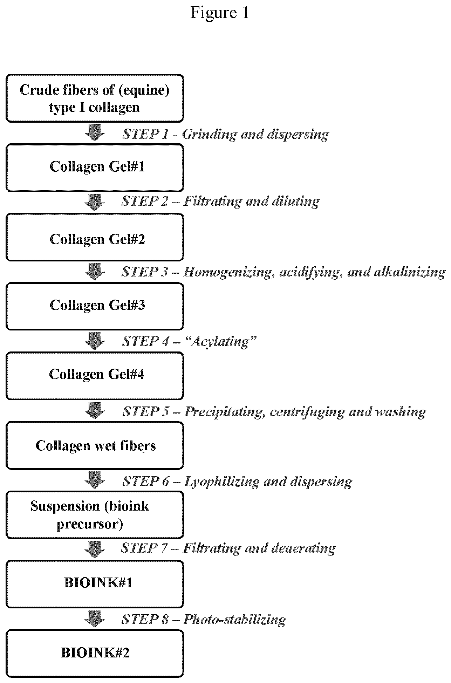

The collagen of the invention may be produced by a multi-step process schematically depicted in Figure 1, from raw fibers of native type I collagen.

According to another of its aspects, subject-matter of the invention is a process for the preparation of the collagen and composition of the invention, comprising the following steps:

- grinding crude fibers of equine type I collagen, dispersing them in an acid solution, filtering the so-obtained suspension gel and diluting it until obtaining a 0.2-0.4% w/v suspension;
- homogenizing the suspension obtained in step (a) at 10,000-20,000 rpm over a period of 1 to 5 minutes at a temperature of 8-20°C, then repeating for a number of cycles from 5 to 20;
- acidifying the suspension obtained in step (b) to a pH of 2.3 to 2.8 at a temperature of 10-25°C;
- alkalinizing the suspension obtained in step (c) to a pH of 8.8 to 9.4 at a temperature of 8-20°C;
- acylating the collagen contained in the suspension obtained in step (d) by addition of a cyclic anhydride at a rate of 10-100 mg/g of collagen, at a pH of 8.8 to 9.2 at a temperature of 8-20°C over a period of 10 minutes to 4 hours;
- precipitating the collagen by addition of an acidic aqueous solution and centrifuging at 8,000-9,000 rpm over a period of 20 to 30 minutes at a temperature of 8-20°C;
- washing the collagen isolated by centrifugation in step (f) by suspending it in water and centrifuging at 8,000-9,000 rpm over a period of 20 to 30 minutes at a temperature of 8-20°C for 4-10 times;
- lyophilizing the collagen isolated by centrifugation in step (g);
- dispersing the lyophilized collagen obtained in step (h) in a solvent at pH 7.0-7.6 to a concentration of 0.5 to 4.0 % w/v, at a temperature of 10-25°C over a period of 5 to 12 hours.

According to a preferred embodiment, suitably hydrated fibers of type I equine collagen are first partially ground (by mild homogenization) in order to increase their specific surface area and the efficiency of the subsequent processing steps, and then dispersed in concentrations ranging from 0.5 % (w/v) to 3.0 % (w/v) in acid solution (pH=3.5±0.5), for an appropriate period under controlled temperature, until a homogeneous viscous gel is obtained. The gel is then filtered, to remove possible residues or fiber agglomerates that are not completely solubilized, and the titer is adjusted until a concentration of 0.2-2.0% (w/v) is obtained. Next, after a further homogenization step, the resulting suspension is cooled and the reaction environment is acidified, preferably with a strong acid, for example an inorganic acid such as hydrochloric acid, and then rapidly basified with a strong base, for example an inorganic base such as a hydroxide of an alkali metal, for example with sodium hydroxide. Next, the acylation of the amine groups of the collagen is carried out, which is achieved by adding a cyclic anhydride to the fibers in an appropriate organic solvent, for example acetone (0.01-0.10 g anhydride per g collagen) and maintaining the pH in the range of 8.5-9.5 for an appropriate period at 8-20°C. The collagen fibers are, then, collected by precipitation by adding an inorganic acid, for example hydrochloric acid, to pH 3.0-4.3 and subsequent centrifugation. Finally, the fibers are washed in purified water to remove the reaction byproducts or possible residues of unreacted materials, are lyophilized and resuspended at the desired concentration (0.5% - 4.0%, w/v) in a pharmaceutically acceptable buffer, for example in phosphate buffered saline (1X) or physiological solution (0.9% w/v sodium chloride). The suspension is finally filtered and may then be subjected to deaeration and transferred to appropriate sterile containers.

Table 1 below shows the reaction conditions in detail, in terms of suitable ranges and preferred embodiments.

Table 1

| Step | Operation | Parameters | Ranges of use | Preferred ranges |
|---|---|---|---|---|
| 1 | Grinding | Speed | 800-6000 rpm | 3000 rpm |
| | | Time | 2-15 min | 8 min |
| | | Temperature | 10-25°C | 15°C |
| | Dispersion | Coll concentration (w/v) | 0.5-3.0% | 1.0% |
| | | | 3.3-3.6 | 3.5 |
| | | pH acid solution | 10-25°C | 15°C |
| | | Temperature | 6-12 h | 8 h |
| | | Time | | |
| 2 | Filtration | Sieve | 35-100 mesh | 60 mesh |
| | | Final concentration | 0.2-2.0% | 0.3% |
| | | Temperature | 20-25°C | 25°C |
| | Dilution | - | - | - |
| 3 | Homogenization | Speed | 10000-20000 rpm | 10000 rpm |
| | | Time | | 2 min |
| | | Temperature | 1-5 min | 10°C |
| | | Number of cycles | 8-20°C | 10 |
| | | | 5-20 | |
| | Acidification | pH | 2.3-2.8 | 2.5 |
| | | Temperature | 10-25°C | 10°C |
| | Alkalinization | pH | 8.8-9.4 | 9.0 |
| | | Temperature | 8-20°C | 10°C |
| 4 | Acylation | Anhydride concentration | 10-100 mg/g coll | 42 mg/g coll 9.0 |
| | | pH | 8.5-9.5 | 10°C |
| | | Temperature | 8-20°C | 30 min |
| | | Time | 10 min - 4h | |
| 5 | Precipitation | pH | 3.9-4.3 | 4.2 |
| | | Temperature | 10-25°C | 10°C |
| | Centrifugation and washing | Speed | 8000-9000 rpm | 8500 rpm |
| | | Time | 20-30 min | 20 min |
| | | Temperature | 8-20°C | 10°C |
| | | Number of repetitions | 4-10 | 6 |
| 6 | Lyophilization | - | - | - |
| | Dispersion | pH | 7.0-7.6 | 7.4 |
| | | Concentration (w/v) | 0.1- 4.0% | 2.5% |
| | | Temperature | 10-25°C | 10°C |
| | | Time | 5-12 h | 6h |
| *- rpm: revolutions per minute; h: hours; min: minutes; coll: collagen* | | | | |

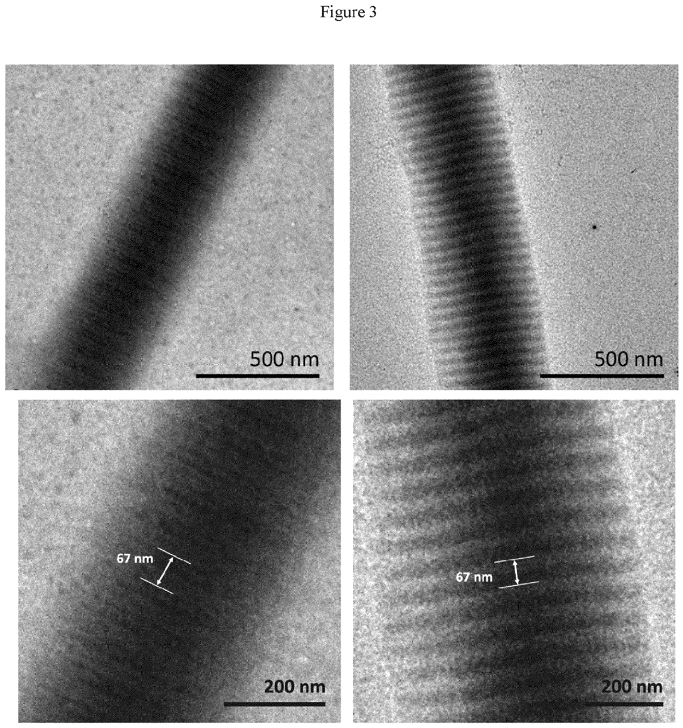

Thanks to the special synthesis protocol used, the resulting composition consisting of only type I collagen in fibrillar form, is characterized by chemical-physical and rheological properties (shown in Figures 3, 4 and 5) that make it particularly suitable for bioprinting applications.

As mentioned, the composition of the invention may be printed as such (with or without other components, for example, selected from other biomaterials, drugs, growth factors, cells and mixtures thereof) or further processed, i.e., subjected to structural stabilization treatments by physical (for example, UV irradiation, heat) or chemical means to increase the stability of the printed construct.

The source of collagen, which is equine-only, has significant advantages, such as lower allergenicity, immunogenicity and very low risk of zoonosis transmission. Furthermore, the high levels of structural organization of this type of collagen (i.e., type I from tendon) allow it not only to provide appropriate physical support to the cells in performing their functions but also to closely mimic the architecture of the extracellular matrix and, therefore, provide mechanical and biochemical stimuli similar to physiological ones.

Furthermore, the composition of the invention offers the possibility to overcome the important disadvantages shown by known collagen bioinks, such as, for example, the narrow operating ranges of viscosity, high sensitivity to temperature and pH, and the need to induce gelling of the material by the use of neutralization buffers. Therefore, the composition of the invention allows to operate at neutral pH and body temperature and without the need for neutralization buffers or chemical crosslinkers. Finally, the composition of the invention is compatible with most popular bioprinting technologies and, in particular but not limited to, all extrusion printing systems.

The collagen obtained in any of steps (f) to (i) of the above described process and Steps 1 to 5 of Table 1, possibly subjected to lyophilization, is a further subject-matter of the invention.

The use of the collagen of the invention in a bioink composition is a further subject-matter of the invention.

Subject-matter of the invention are also a 3D printing method that comprises printing a composition of the invention, preferably but not only, by extrusion of said composition.

The collagen and composition of the invention can be used in therapy and surgery, for example, the use in 3D printing scaffolds for tissue engineering and/or regenerative medicine applications for hard and soft tissue regeneration, dressings and 3D cellular models for *in vitro* studying physiological, pathological and tumor processes and the like.

## Experimental section

### Example 1

### Example 1a. Preparation of the collagen of the invention

Crude fibers of native, type I collagen, in the wet state are partially ground by mild homogenization at 3000 rpm, at a temperature of 15°C for 8 min. Next, they are dispersed at 1% (w/v) in a 0.5 M acetic acid aqueous solution (pH 3.5) for 6-8 hours at controlled temperature (10-15°C) until a homogenous viscous gel is obtained. At this point, the gel is filtered through a 60-mesh sieve and the titer adjusted until a 0.3 percent (w/v) suspension is obtained. Next, the suspension (e.g., 100 ml) is subjected to 10 cycles of homogenization at 10000 rpm for 2 min, interspersed with 5 min pauses, at controlled temperature (10-15°C). Next, acidification of the reaction environment to pH 2.5 is carried out with hydrochloric acid, and then the pH is rapidly raised to 9.0 with sodium hydroxide (T=10°C). At this point, the acylation is performed by adding 14 mg glutaric anhydride previously dissolved in 1 ml acetone (0.042 g anhydride per g collagen). In this step, the reaction pH is constantly monitored and kept constant and equal to about 9.0 (8.8-9.2) by addition of sodium hydroxide for the whole duration of the acylation (30 minutes) at 10-15°C. The collagen fibers are then collected by precipitation with hydrochloric acid to pH 4.2 and then collected by centrifugation at 8500 rpm for 20 minutes at 10°C, resulting in the collagen of the invention. The fibers are then washed in purified water (6 washes performed by resuspension of the pellet (constituted by the collagen fibers) in purified water and centrifugation at 8500 rpm for 20 minutes at 10°C) and lyophilized, thus giving the lyophilized collagen of the invention.

### Example 1b. Preparation of the composition of the invention

The composition of the invention is obtained by dispersion (6 hours, 10°C) of the lyophilized fibers in phosphate buffered saline (1X), or physiological solution (0.9% sodium chloride) or cell culture medium at the desired concentration, preferably 2.5% (w/v), followed by filtration with a 100-mesh sieve and deaeration and possible final photo-stabilization.

## Claims

1. A bioink composition comprising equine type I collagen, **characterized in that**

   - the pH of said composition is from 7.0 to 7.6;
   - said composition is pseudoplastic; and
   - said equine type I collagen is in suspension and has a weight average molecular weight of the $\alpha_1$(I) and $\alpha_2$(I)

chains ranging from 120 to 200 kDa.

2. The composition according to claim 1, **characterized in that** said collagen is in suspension in a solvent selected from a pharmaceutically acceptable buffer, preferably saline phosphate buffer, and a 0.9% w/v sodium chloride physiological solution.

3. The composition according to claim 1 or 2, **characterized in that** said collagen is present in the composition at a concentration of 0.5 to 4% w/v.

4. The composition according to any one of claims 1 to 3, **characterized in that** it also comprises at least one further biological agent, preferably selected from drugs, growth factors, proteins, cells and mixtures thereof.

5. The composition according to any one of claims 1 to 4, **characterized in that** the viscosity $\eta$ is between 1 and 10000 Pa*s, for deformation rate equal to 0.1 Hz, and between 0.01 and 100 Pa*s for deformation rate equal to 100 Hz, preferably the viscosity $\eta$ is between 5 and 5000 Pa*s, for deformation rate equal to 0.1 Hz, and between 0.1 and 10 Pa*s for deformation rate equal to 100 Hz.

6. The composition according to any one of claims 1 to 4, **characterized in that** the storage modulus G' is between 1 and 10000 Pa for frequencies equal to 0.1 Hz, and between 10 and 10000 Pa for frequencies equal to 10 Hz, and the loss modulus G" is between 0.1 and 1000 Pa for frequencies equal to 0.1 Hz , and between 1 and 1000 Pa for frequencies equal to 10 Hz, preferably the storage modulus G' is between 5 and 5000 Pa for frequencies equal to 0.1 Hz, and between 50 and 5000 Pa for frequencies equal to 10 Hz, and the loss modulus G" is between 0.5 and 500 Pa for frequencies equal to 0.1 Hz, and between 5 and 500 Pa for frequencies equal to 10 Hz.

7. The composition according to any one of claims 1 to 4, **characterized in that** said composition has G' > G" and G"/G' ratio between 0.1 and 0.3.

8. The composition according to any one of claims 1 to 4, **characterized in that** said equine type I collagen is in fibrillar form and exhibits the typical banding pattern of the native collagen with a period of 67 nm.

9. The composition according to any one of claims 1 to 8, for its use in therapy and surgery, preferably for 3D printing scaffolds for tissue engineering and/or regenerative medicine applications for hard and soft tissue regeneration, dressings and 3D cellular models for *in vitro* studying physiological, pathological and tumor processes.

10. A process for the preparation of the composition of any one of claims 1 to 8, comprising the following steps of:

   a. grinding crude fibers of equine type I collagen, dispersing them in an acid solution, filtering the so-obtained suspension gel and diluting it until obtaining a 0.2-0.4% w/v suspension;
   b. homogenizing the suspension obtained in step (a) at 10,000-20,000 rpm over a period of 1 to 5 minutes at a temperature of 8-20°C, then repeating for a number of cycles from 5 to 20;
   c. acidifying the suspension obtained in step (b) to a pH of 2.3 to 2.8 at a temperature of 10-25°C;
   d. alkalinizing the suspension obtained in step (c) to a pH of 8.8 to 9.4 at a temperature of 8-20°C;
   e. acylating the collagen contained in the suspension obtained in step (d) by addition of a cyclic anhydride at a rate of 10-100 mg/g of collagen, at a pH of 8.8 to 9.2 at a temperature of 8-20°C over a period of 10 minutes to 4 hours;
   f. precipitating the collagen by addition of an acidic aqueous solution and centrifuging at 8,000-9,000 rpm over a period of 20 to 30 minutes at a temperature of 8-20°C;
   g. washing the collagen isolated by centrifugation in step (f) by suspending it in water and centrifuging at 8,000-9,000 rpm over a period of 20 to 30 minutes at a temperature of 8-20°C for 4-10 times;
   h. lyophilizing the collagen isolated by centrifugation in step (g);
   i. dispersing the lyophilized collagen obtained in step (h) in a solvent at pH 7.0-7.6 to a concentration of 0.5 to 4.0 % w/v, at a temperature of 10-25°C over a period of 5 to 12 hours.

11. The process according to claim 10, **characterized in that** the cyclic anhydride of step (e) is selected from succinic anhydride and glutaric anhydride.

12. A composition prepared by a process comprising the following steps:

a. grinding crude fibers of equine type I collagen, dispersing them in an acid solution, filtering the so-obtained suspension gel and diluting it until obtaining a 0.2-0.4% w/v suspension;

b. homogenizing the suspension obtained in step (a) at 10,000-20,000 rpm over a period of 1 to 5 minutes at a temperature of 8-20°C, then repeating for a number of cycles from 5 to 20;

c. acidifying the suspension obtained in step (b) to a pH of 2.3 to 2.8 at a temperature of 10-25°C;

d. alkalinizing the suspension obtained in step (c) to a pH of 8.8 to 9.4 at a temperature of 8-20°C;

e. acylating the collagen contained in the suspension obtained in step (d) by addition of a cyclic anhydride at a rate of 10-100 mg/g of collagen, at a pH of 8.8 to 9.2 at a temperature of 8-20°C over a period of 10 minutes to 4 hours;

f. precipitating the collagen by addition of an acidic aqueous solution and centrifuging at 8,000-9,000 rpm over a period of 20 to 30 minutes at a temperature of 8-20°C;

g. optionally washing the collagen isolated by centrifugation in step (f) by suspending it in water and centrifuging at 8,000-9,000 rpm over a period of 20 to 30 minutes at a temperature of 8-20°C for 4-10 times;

h. optionally lyophilizing the collagen isolated by centrifugation in step (g);

i. optionally dispersing the lyophilized collagen obtained in step (h) in a solvent at pH 7.0-7.6 to a concentration of 0.5 to 4.0 % w/v, at a temperature of 10-25°C over a period of 5 to 12 hours;

optionally wherein the cyclic anhydride of step (e) is selected from succinic anhydride and glutaric anhydride.

**13.** A 3D printing method comprising printing a composition according to any one of claims 1 to 8.

**14.** The method according to claim 13, **characterized in that** it comprises extruding said composition.

**Patentansprüche**

**1.** Eine Biotintenzusammensetzung, die Kollagen vom Typ I von Pferden enthält, **dadurch gekennzeichnet, dass**

- der pH-Wert der Zusammensetzung zwischen 7,0 und 7,6 liegt;
- die Zusammensetzung pseudoplastisch ist; und
- das Kollagen vom Typ I von Pferden in Suspension vorliegt und ein gewichtsdurchschnittliches Molekulargewicht der $\alpha_1$(I)- und $\alpha_2$(I)-Ketten im Bereich von 120 bis 200 kDa aufweist.

**2.** Die Zusammensetzung gemäß Anspruch 1, **dadurch gekennzeichnet, dass** das Kollagen in einem Lösungsmittel suspendiert ist, das aus einem pharmazeutisch verträglichen Puffer, vorzugsweise einem Salzphosphatpuffer, und einer 0,9 % (Gew./Vol.) physiologischen Natriumchloridlösung ausgewählt ist.

**3.** Die Zusammensetzung gemäß Anspruch 1 oder 2, **dadurch gekennzeichnet, dass** das Kollagen in der Zusammensetzung in einer Konzentration von 0,5 bis 4 % (Gew./Vol.) vorhanden ist.

**4.** Die Zusammensetzung nach einem der Ansprüche 1 bis 3, **dadurch gekennzeichnet, dass** sie außerdem mindestens einen weiteren biologischen Wirkstoff enthält, der vorzugsweise aus Arzneimitteln, Wachstumsfaktoren, Proteinen, Zellen und Mischungen davon ausgewählt ist.

**5.** Die Zusammensetzung gemäß einem der Ansprüche 1 bis 4, **dadurch gekennzeichnet, dass** die Viskosität $\eta$ zwischen 1 und 10000 Pa*s bei einer Deformationsrate von 0,1 Hz und zwischen 0,01 und 100 Pa*s bei einer Deformationsrate von 100 Hz liegt, vorzugsweise liegt die Viskosität $\eta$ zwischen 5 und 5000 Pa*s bei einer Deformationsrate von 0,1 Hz und zwischen 0,1 und 10 Pa*s bei einer Deformationsrate von 100 Hz.

**6.** Die Zusammensetzung nach einem der Ansprüche 1 bis 4, **dadurch gekennzeichnet, dass** der Speichermodul G' zwischen 1 und 10000 Pa für Frequenzen gleich 0,1 Hz und zwischen 10 und 10000 Pa für Frequenzen gleich 10 Hz liegt und der Verlustmodul G" zwischen 0,1 und 1000 Pa für Frequenzen gleich 0,1 Hz und zwischen 1 und 1000 Pa für Frequenzen gleich 10 Hz liegt, vorzugsweise liegt der Speichermodul G' zwischen 5 und 5000 Pa für Frequenzen gleich 0,1 Hz und zwischen 50 und 5000 Pa für Frequenzen gleich 10 Hz und der Verlustmodul G" zwischen 0,5 und 500 Pa für Frequenzen gleich 0,1 Hz und zwischen 5 und 500 Pa für Frequenzen gleich 10 Hz liegt.

**7.** Die Zusammensetzung gemäß einem der Ansprüche 1 bis 4, **dadurch gekennzeichnet, dass** die Zusammensetzung G'>G" und ein G"/G'-Verhältnis zwischen 0,1 und 0,3 aufweist.

8. Die Zusammensetzung nach einem der Ansprüche 1 bis 4, **dadurch gekennzeichnet, dass** das Kollagen vom Typ I von Pferden in fibrillärer Form vorliegt und das typische Bandenmuster des nativen Kollagens mit einer Periode von 67 nm aufweist.

9. Die Zusammensetzung gemäß einem der Ansprüche 1 bis 8 für ihre Verwendung in Therapie und Chirurgie, vorzugsweise für den 3D-Druck von Gerüsten für Anwendungen im Tissue Engineering und/oder der regenerativen Medizin zur Regeneration von Hart- und Weichgewebe, Verbänden und 3D-Zellmodellen für die In-vitro-Untersuchung physiologischer, pathologischer und Tumorprozesse.

10. Ein Verfahren zur Herstellung der Zusammensetzung nach einem der Ansprüche 1 bis 8, das die folgenden Schritte umfasst:

   a. Mahlen von Rohfasern aus Kollagen vom Typ I von Pferden, deren Dispergieren in einer Säurelösung, Filtern des so erhaltenen Suspensionsgels und Verdünnen desselben bis eine 0,2 bis 0,4 % (Gew./Vol.) Suspension erhalten wird;
   b. Homogenisieren der in Schritt (a) erhaltenen Suspension bei 10.000 bis 20.000 U/min über einen Zeitraum von 1 bis 5 Minuten bei einer Temperatur von 8 bis 20 °C, dann Wiederholen für eine Anzahl von Zyklen von 5 bis 20;
   c. Ansäuern der in Schritt (b) erhaltenen Suspension auf einen pH-Wert von 2,3 bis 2,8 bei einer Temperatur von 10 bis 25 °C;
   d. Alkalisieren der in Schritt (c) erhaltenen Suspension auf einen pH-Wert von 8,8 bis 9,4 bei einer Temperatur von 8 bis 20 °C;
   e. Acylieren des in der in Schritt (d) erhaltenen Suspension enthaltenen Kollagens durch Zugabe eines zyklischen Anhydrids in einer Menge von 10 bis 100 mg/g Kollagen, bei einem pH-Wert von 8,8 bis 9,2 bei einer Temperatur von 8 bis 20°C über einen Zeitraum von 10 Minuten bis 4 Stunden;
   f. Ausfällen des Kollagens durch Zugabe einer sauren wässrigen Lösung und Zentrifugieren bei 8.000 bis 9.000 U/min über einen Zeitraum von 20 bis 30 Minuten bei einer Temperatur von 8 bis 20 °C;
   g. optional Waschen des in Schritt (f) durch Zentrifugation isolierten Kollagens durch Suspendieren in Wasser und 4- bis 10-maliges Zentrifugieren bei 8.000 bis 9.000 U/min über einen Zeitraum von 20 bis 30 Minuten bei einer Temperatur von 8 bis 20 °C;
   h. optional Gefriertrocknung des in Schritt (g) durch Zentrifugation isolierten Kollagens;
   i. optional Dispergieren des in Schritt (h) erhaltenen gefriergetrockneten Kollagens in einem Lösungsmittel mit einem pH-Wert von 7,0 bis 7,6 auf eine Konzentration von 0,5 bis 4,0 % (Gew./Vol.) bei einer Temperatur von 10 bis 25 °C über einen Zeitraum von 5 bis 12 Stunden.

11. Das Verfahren nach Anspruch 10, **dadurch gekennzeichnet, dass** das zyklische Anhydrid von Schritt (e) aus Bernsteinsäureanhydrid und Glutarsäureanhydrid ausgewählt ist.

12. Eine Zusammensetzung hergestellt nach dem Verfahren, umfassend die folgenden Schritte:

   a. Mahlen von Rohfasern aus Kollagen vom Typ I von Pferden, deren Dispergieren in einer Säurelösung, Filtern des so erhaltenen Suspensionsgels und Verdünnen desselben, bis eine 0,2 bis 0,4 % (Gew./Vol.) Suspension erhalten wird;
   b. Homogenisieren der in Schritt (a) erhaltenen Suspension bei 10.000 bis 20.000 U/min über einen Zeitraum von 1 bis 5 Minuten bei einer Temperatur von 8 bis 20 °C, dann Wiederholen für eine Anzahl von Zyklen von 5 bis 20;
   c. Ansäuern der in Schritt (b) erhaltenen Suspension auf einen pH-Wert von 2,3 bis 2,8 bei einer Temperatur von 10 bis 25 °C;
   d. Alkalisieren der in Schritt (c) erhaltenen Suspension auf einen pH-Wert von 8,8 bis 9,4 bei einer Temperatur von 8 bis 20 °C;
   e. Acylieren des in der in Schritt (d) erhaltenen Suspension enthaltenen Kollagens durch Zugabe eines zyklischen Anhydrids in einer Menge von 10 bis 100 mg/g Kollagen, bei einem pH-Wert von 8,8 bis 9,2 bei einer Temperatur von 8 bis 20°C über einen Zeitraum von 10 Minuten bis 4 Stunden;
   f. Ausfällen des Kollagens durch Zugabe einer sauren wässrigen Lösung und Zentrifugieren bei 8.000 bis 9.000 U/min über einen Zeitraum von 20 bis 30 Minuten bei einer Temperatur von 8 bis 20 °C;
   g. Waschen des in Schritt (f) durch Zentrifugation isolierten Kollagens durch Suspendieren in Wasser und 4- bis 10-maliges Zentrifugieren bei 8.000 bis 9.000 U/min über einen Zeitraum von 20 bis 30 Minuten bei einer Temperatur von 8 bis 20 °C;

h. Gefriertrocknung des in Schritt (g) durch Zentrifugation isolierten Kollagens;

i. Dispergieren des in Schritt (h) erhaltenen gefriergetrockneten Kollagens in einem Lösungsmittel mit einem pH-Wert von 7,0 bis 7,6 auf eine Konzentration von 0,5 bis 4,0 % w/v bei einer Temperatur von 10 bis 25 °C über einen Zeitraum von 5 bis 12 Stunden;

wobei optional das zyklische Anhydrid von Schritt (e) aus Bernsteinsäureanhydrid und Glutarsäureanhydrid ausgewählt ist.

**13.** Ein 3D-Druckverfahren, das das Drucken einer Zusammensetzung gemäß einem der Ansprüche 1 bis 8 umfasst.

**14.** Das Verfahren gemäß Anspruch 13, **dadurch gekennzeichnet, dass** es das Extrudieren der Zusammensetzung umfasst.

**Revendications**

**1.** Composition de bioencre comprenant du collagène équin de type I, **caractérisée en ce que**

- le pH de ladite composition est compris entre 7,0 et 7,6 ;
- ladite composition est pseudoplastique ; et
- ledit collagène équin de type I est en suspension et a un poids moléculaire moyen en poids des chaînes $\alpha_1(I)$ et $\alpha_2(I)$ compris entre 120 et 200 kDa.

**2.** Composition selon la revendication 1, **caractérisée en ce que** ledit collagène est en suspension dans un solvant choisi parmi un tampon pharmaceutiquement acceptable, de préférence un tampon phosphate salin, et une solution physiologique de chlorure de sodium à 0,9 % p/v.

**3.** Composition selon la revendication 1 ou 2, **caractérisée en ce que** ledit collagène est présent dans la composition à une concentration de 0,5 à 4% p/v.

**4.** Composition selon l'une quelconque des revendications 1 à 3, **caractérisée en ce qu'**elle comprend également au moins un autre agent biologique, de préférence choisi parmi les médicaments, les facteurs de croissance, les protéines, les cellules et leurs mélanges.

**5.** Composition selon l'une quelconque des revendications 1 à 4, **caractérisée en ce que** la viscosité $\eta$ est comprise entre 1 et 10000 Pa*s, pour une vitesse de déformation égale à 0,1 Hz, et entre 0,01 et 100 Pa*s pour une vitesse de déformation égale à 100 Hz, de préférence la viscosité $\eta$ est comprise entre 5 et 5000 Pa*s, pour une vitesse de déformation égale à 0,1 Hz, et entre 0,1 et 10 Pa*s pour une vitesse de déformation égale à 100 Hz.

**6.** Composition selon l'une quelconque des revendications 1 à 4, **caractérisée en ce que** le module de stockage G' est compris entre 1 et 10000 Pa pour des fréquences égales à 0,1 Hz, et entre 10 et 10000 Pa pour des fréquences égales à 10 Hz, et le module de perte G" est compris entre 0,1 et 1000 Pa pour des fréquences égales à 0,1 Hz, et entre 1 et 1000 Pa pour des fréquences égales à 10 Hz, de préférence le module de stockage G' est compris entre 5 et 5000 Pa pour des fréquences égales à 0,1 Hz, et entre 50 et 5000 Pa pour des fréquences égales à 10 Hz, et le module de perte G" est compris entre 0,5 et 500 Pa pour des fréquences égales à 0,1 Hz, et entre 5 et 500 Pa pour des fréquences égales à 10 Hz.

**7.** Composition selon l'une quelconque des revendications 1 à 4, **caractérisée en ce que** ladite composition présente un rapport G' > G" et un rapport G"/G' compris entre 0,1 et 0,3.

**8.** Composition selon l'une quelconque des revendications 1 à 4, **caractérisée en ce que** le collagène équin de type I est sous forme fibrillaire et présente le motif de bande typique du collagène natif avec une période de 67 nm.

**9.** Composition selon l'une quelconque des revendications 1 à 8, pour son utilisation en thérapie et en chirurgie, de préférence pour l'impression 3D d'échafaudages pour l'ingénierie tissulaire et/ou les applications de médecine régénérative pour la régénération des tissus durs et mous, les pansements et les modèles cellulaires 3D pour l'étude *in vitro* des processus physiologiques, pathologiques et tumoraux.

**10.** Procédé de préparation de la composition de l'une quelconque des revendications 1 à 8, comprenant les étapes suivantes consistant à :

a. broyer des fibres brutes de collagène équin de type I, les disperser dans une solution acide, filtrer le gel de suspension ainsi obtenu et le diluer jusqu'à l'obtention d'une suspension de 0,2 à 0,4 % p/v ;

b. homogénéiser la suspension obtenue à l'étape (a) à 10 000 à 20 000 tours/minute pendant une période de 1 à 5 minutes à une température de 8 à 20°C, puis répéter pour un nombre de cycles de 5 à 20 ;

c. acidifier la suspension obtenue à l'étape (b) jusqu'à un pH de 2,3 à 2,8 à une température de 10 à 25°C ;

d. alcaliniser la suspension obtenue à l'étape (c) à un pH de 8,8 à 9,4 à une température de 8 à 20°C;

e. acyler la collagène contenu dans la suspension obtenue à l'étape (d) par addition d'un anhydride cyclique à raison de 10 à 100 mg/g de collagène, à un pH de 8,8 à 9,2 et à une température de 8 à 20°C pendant une période de 10 minutes à 4 heures ;

f. précipiter le collagène en ajoutant une solution aqueuse acide et en centrifugeant à 8 000 à 9 000 tours/minute pendant une période de 20 à 30 minutes à une température de 8 à 20°C ;

g. laver le collagène isolé par centrifugation à l'étape (f) en le suspendant dans de l'eau et en le centrifugeant à 8 000 à 9 000 tours/minute pendant une période de 20 à 30 minutes à une température de 8 à 20 °C pendant 4 à 10 fois ;

h. lyophiliser le collagène isolé par centrifugation à l'étape (g) ;

i. disperser le collagène lyophilisé obtenu à l'étape (h) dans un solvant à un pH de 7,0 à 7,6 à une concentration de 0,5 à 4,0 % p/v, à une température de 10 à 25°C pendant une période de 5 à 12 heures.

**11.** Procédé selon la revendication 10, **caractérisé en ce que** l'anhydride cyclique de l'étape (e) est choisi parmi l'anhydride succinique et l'anhydride glutarique.

**12.** Composition préparée par un procédé comprenant les étapes suivantes consistant à :

a. broyer des fibres brutes de collagène équin de type I, les disperser dans une solution acide, filtrer le gel de suspension ainsi obtenu et le diluer jusqu'à l'obtention d'une suspension de 0,2 à 0,4 % p/v ;

b. homogénéiser la suspension obtenue à l'étape (a) à 10 000 à 20 000 tours/minute pendant une période de 1 à 5 minutes à une température de 8 à 20°C, puis répéter pour un nombre de cycles de 5 à 20 ;

c. acidifier la suspension obtenue à l'étape (b) jusqu'à un pH de 2,3 à 2,8 à une température de 10 à 25°C;

d. alcaliniser la suspension obtenue à l'étape (c) à un pH de 8,8 à 9,4 à une température de 8 à 20°C;

e. acyler le collagène contenu dans la suspension obtenue à l'étape (d) par addition d'un anhydride cyclique à raison de 10 à 100 mg/g de collagène, à un pH de 8,8 à 9,2 et à une température de 8 à 20°C pendant une période de 10 minutes à 4 heures ;

f. précipiter le collagène en ajoutant une solution aqueuse acide et en centrifugeant à 8 000 à 9 000 tours/minute pendant une période de 20 à 30 minutes à une température de 8 à 20°C ;

g. éventuellement, laver le collagène isolé par centrifugation à l'étape (f) en le suspendant dans de l'eau et en le centrifugeant à 8 000 à 9 000 tours/minute pendant une période de 20 à 30 minutes à une température de 8 à 20 °C pendant 4 à 10 fois ;

h. éventuellement, lyophiliser le collagène isolé par centrifugation à l'étape g) ;

i. éventuellement, disperser le collagène lyophilisé obtenu à l'étape (h) dans un solvant à un pH de 7,0 à 7,6 à une concentration de 0,5 à 4,0 % p/v, à une température de 10 à 25°C pendant une période de 5 à 12 heures ;

éventuellement dans lequel l'anhydride cyclique de l'étape (e) est choisi parmi l'anhydride succinique et l'anhydride glutarique.

**13.** Procédé d'impression 3D comprenant l'impression d'une composition selon l'une quelconque des revendications 1 à 8.

**14.** Procédé selon la revendication 13, **caractérisée en ce qu'**elle comprend l'extrusion de ladite composition.

Figure 1

```
┌─────────────────────────┐
│  Crude fibers of (equine)│
│     type I collagen      │
└─────────────────────────┘
         ⬇   STEP 1 - Grinding and dispersing
┌─────────────────────────┐
│     Collagen Gel#1       │
└─────────────────────────┘
         ⬇   STEP 2 – Filtrating and diluting
┌─────────────────────────┐
│     Collagen Gel#2       │
└─────────────────────────┘
         ⬇   STEP 3 – Homogenizing, acidifying, and alkalinizing
┌─────────────────────────┐
│     Collagen Gel#3       │
└─────────────────────────┘
         ⬇   STEP 4 – "Acylating"
┌─────────────────────────┐
│     Collagen Gel#4       │
└─────────────────────────┘
         ⬇   STEP 5 – Precipitating, centrifuging and washing
┌─────────────────────────┐
│    Collagen wet fibers   │
└─────────────────────────┘
         ⬇   STEP 6 – Lyophilizing and dispersing
┌─────────────────────────┐
│    Suspension (bioink    │
│        precursor)        │
└─────────────────────────┘
         ⬇   STEP 7 – Filtrating and deaerating
┌─────────────────────────┐
│        BIOINK#1          │
└─────────────────────────┘
         ⬇   STEP 8 – Photo-stabilizing
┌─────────────────────────┐
│        BIOINK#2          │
└─────────────────────────┘
```

Figure 2

Figure 3

Figure 4

Figure 5

Figure 6

Figure 7

EP 4 249 008 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NOCERA ADEN DIAZ et al.** Development of 3D printed fibrillar collagen scaffold for tissue engineering. *BIOMEDICAL MICRODEVICES, SPRINGER US, NEW YORK,* 27 February 2018, vol. 20 (2), 1-13

- Rheology. *Guidelines for standardization of bioprinting: a systematic study of process parameters and their effect on bioprinted structures,* 10